# EUROPÄISCHE PATENTSCHRIFT

(11) **EP 2 750 644 B1**
(45) Veröffentlichungstag und Bekanntmachung des Hinweises auf die Patenterteilung: **10.10.2018**
(21) Anmeldenummer: 12762555.6
(22) Anmeldetag: 29.08.2012
(51) Int. Cl.: A61K 9/70, A61F 13/02, A61L 15/44, A61L 15/58, A61K 31/085, A61K 31/165, A61K 31/205, A61K 31/522

(54) **WIRKSTOFFHALTIGE HAUTAUFLAGEN**
DRESSINGS THAT CONTAIN ACTIVE SUBSTANCE
PANSEMENT CONTENANT DES PRINCIPES ACTIFS

(30) Priorität: 30.08.2011 DE 102011081818
(43) Veröffentlichungstag der Anmeldung: 09.07.2014
(60) Teilanmeldung: 18170007.1
(73) Patentinhaber: Beiersdorf AG, 20253 Hamburg (DE)
(72) Erfinder: NIERLE, Jens, 21073 Hamburg (DE); WÖLLER, Karl-Heinz, 20257 Hamburg (DE); ZIER, Maxi, 23701 Eutin (DE); MÜLLER, Gerd, 22397 Hamburg (DE)
(74) Vertreter: Hartmann, Jost
(86) Internationale Anmeldenummer: PCT/EP2012/066768
(87) Internationale Veröffentlichungsnummer: WO 2013/030227

(56) Entgegenhaltungen:
- GB-A- 781 975
- GB-A- 1 570 669
- GB-A- 2 063 102
- GB-A- 2 425 487
- US-A1- 2009 182 256
- US-A1- 2010 298 747

## Beschreibung

Die selbstklebenden Hautauflagen umfassen in eine Richtung dehnbares und rückstellfähiges Trägermaterial auf dem in streifen- und/oder wellenform Klebemassen quer zur Dehnungsrichtung aufgetragen sind, die ein oder mehrere Wirkstoffe enthalten.

Wärmende Produkte spielen in der Behandlung von muskulären Schmerzen schon seit vielen Jahren eine bedeutende Rolle. So gibt es seit 1928 das ABC-Pflaster® mit Capsaicinoiden als Wirkstoffe. Neben diesem Pflaster sind diverse weitere Produkte auf dem Markt die Wirkstoffe mit Capsaicinoiden oder Derivaten davon verwenden.

Die Wirkweise dieser Produkte beruht zum einen auf der Förderung der Durchblutung in den betroffenen Arealen und zum Anderen auf einen Einfluss auf den Stoffwechsel. So wird zum Beispiel durch Capsaicin die Ausschüttung von Substanz P, ein Neuropeptid aus elf Aminosäuren, angeregt. Dieses Molekül greift aktiv in den Schmerzkreislauf ein und trägt zu einer Verminderung der wahrgenommenen Schmerzen bei.

Bei Cellulite (medizinischer Begriff: Dermopanniculosis) handelt es sich nicht um eine Krankheit, sondern um ein kosmetisches Problem.

Die Ursachen für Cellulite liegen vor allem am speziellen Aufbau der weiblichen Haut und an der Reaktion auf die weiblichen Hormone. In der Unterhaut sind Fettzellen gespeichert. Deren Menge wird schon im Säuglingsstadium festgelegt und ist durch Ernährung oder Sport nicht beeinflussbar. In den Fettzellen werden die Fettsäuren aus der Nahrung in Fette umgewandelt und knötchenförmig ins Bindegewebe eingelagert. Wenn diese Fette über längere Zeit nicht abgebaut werden (z.B. Sport), und wird der Körper zusätzlich überernährt, können sich die Zellen um ein Vielfaches Ihrer Größe ausdehnen. Die vergrößerten Zellen drücken sich dann durch das Bindegewebe und es kommt zur gefürchteten Orangenhaut, auch als Cellulite bezeichnet.

Die weiteren Folgen im Alter sind Besenreiser, Krampfadern, Thrombosen und Beinleiden. Oft sind die Oberschenkel auch Speicher für überflüssiges Fett, dass über die Nahrung aufgenommen wird. Die unschönen, seitlichen Verdickungen der Oberschenkel, auch Reiterhosen genannt, kombiniert mit Cellulitis stellen oft eine große Belastung für die Betroffene dar.

Lästiges kosmetisches Phänomen sind Streifen in der Haut nach der Schwangerschaft, die im ästhetischen Erscheinungsbild störend wirken. Sogenannte Dehnungsstreifen werden auch Stria oder Schwangerschaftsstreifen genannt. Es handelt sich auch hier nicht unbedingt um eine Krankheit, sondern um ein rein kosmetisches Problem.

Hautdehnungsstreifen (Stria) sind Risse im Unterhautgewebe. Sie entstehen an Bauch, Hüften oder Brust. Striae sind erst bläulich-rot, später gelblich-weiß. Sie besitzen ein ähnliches Aussehen wie Narben. Sie entstehen, wenn die Haut überdehnt wird und zugleich die Dehnungsfähigkeit der Haut nachgelassen hat. Ein hoher Cortisonspiegel fördert die Bildung der Dehnungsstreifen. Dieses Hormon lässt die Haut mehr Wasser festhalten und es vermindert die Elastizität der Haut.

Wird die Haut durch eine Schwangerschaft oder Gewichtszunahme gedehnt, entstehen kleine Risse im elastischen Gewebe. Die Haut wird an den betreffenden Stellen dünner und die Blutgefäße schimmern bläulich durch. Später vernarben die Stellen und die Streifen werden weiß. Bedauerlicherweise ist auf ein Verschwinden der Streifen nicht zu hoffen. Schwangere Personen, Personen in der Pubertät, Leistungssportler, Personen in einer Hormonbehandlung und Personen mit erhöhtem Körpergewicht sind die überwiegend von Stria betroffenen Personengruppen.

In der Schwangerschaft ist der Cortisonspiegel im Blut erhöht. Hier entstehen bei vielen Frauen Dehnungsstreifen in der Bauchhaut. Man nennt sie "Striae gravidarum" oder Schwangerschaftsstreifen.

Sind Dehnungsstreifen vorhanden, so können sie nach heutigem Wissenstand nicht mehr vollständig reduziert werden. Eine Reduzierung und Linderung bis zu 50 % ist jedoch möglich. Auch für Laserbehandlungen gilt, dass sie meist nicht den gewünschten Erfolg bringen.

Aufgabe der vorliegenden Erfindung ist es daher eine Hautauflage bereit zu stellen, die eine Behandlung der von Stria und Cellulite betroffenen Hautpartien ermöglicht und eine kosmetische Verbesserung dieser Hautpartien bewirkt.

DE 10056009 A1 offenbart wirkstoffhaltige Matrixpflaster zur kontrollierten Abgabe von hyperämisierenden Wirkstoffen.

DE 19650471 A1 beschreibt wirkstoffhaltige Pflaster mit einem Trägermaterial und einer darauf aufgebrachten Heißschmelzklebemasse, die mindestens einen hyperämisierenden Wirkstoff enthält. Die Klebmasse kann vollflächig oder partiell aufgetragen sein, letzteres um die Luft- und Wasserdampfdurchlässigkeit zu verbessern. Zu Dehnbarkeit, Kinesio oder speziellen Klebmassenbeschichtungen schweigt die Druckschrift.

DE 19749467 A1 beschreibt wirkstoffhaltige Pflaster mit einem Trägermaterial und einer darauf aufgebrachten Heißschmelzklebemasse, die mindestens einen Wirkstoff enthält, wobei die Klebmasse geschäumt ist.

Erwähnt wird weiterhin, dass es zu keiner Verschiebung von Hautlagen aufgrund der geschäumten Klebmasse kommt.

DE 19804604 A1 offenbart Vorrichtungen zur Freigabe von Stoffen, wobei die Vorrichtungen eine Klebmasse mit SEPS (Styrol-Ethylen-Propylen-Styrol) - Blockcopolymeren umfasst. Zu Dehnbarkeit, Kinesio oder speziellen Klebmassenbeschichtungen schweigt die Druckschrift.

Die DE 19804774 A1 offenbart Trägermaterialien für medizinische Zwecke, wobei das Trägermaterial ein mittels Nähfäden übernähtes Vlies mit einer Höchstzugkraft von mindestens 10 N/cm umfasst. Die auf dem Träger aufgebrachte Klebmasse kann Wirkstoffe beinhalten. Das Trägermaterial erzeugt bei einer Dehnung von 20 bis 70% eine Kompressionskraft von 0,2 bis 10 N/cm.

DE 19825499 A1 offenbart wirkstoffhaltige Pflaster, wobei die Klebmasse in Form von versponnenen Fasern oder Fäden, also in ungeordneter Form, aufgebracht ist.

DE 10012582 A1 beschreibt ein Verfahren zum Auftragen von Klebmasse auf Trägermaterialien, wobei der Auftrag in Wellenform in Richtung der Längsrichtung des Trägers erfolgt.

Neben den klassischen Schmerzpräparaten haben sich in den letzten Jahren wirkstofffreie Behandlungsmethoden wie zum Beispiel Kinesio etabliert.

In US 5861348 wird erstmals die Kinesiotherapie beschrieben und beansprucht. Wichtig und Grundlage ist dabei der wellenförmige Auftrag einer Klebmasse ohne Wirkstoffbeeinflussung.

Hier beruht der Behandlungseffekt in erster Linie auf einer mechanischen Wirkung. Das zu behandelnde Hautareal wird einer gewissen Vorspannung ausgesetzt und dann mit einem Streifen des elastischen Kinesiotapes beklebt. Wenn sich nun die Haut des behandelten Areals entspannt wirft das Tape Falten die quer zur Laufrichtung des Tapes ausgerichtet sind. Durch die Kombination dieser Falten mit den normalen Bewegungen der Haut wird eine ständige Reizwirkung auf die betroffene Stelle ausgeübt, die die Muskelverspannungen bzw. Schmerzen positiv beeinflusst.

Durch die Haftung des Tapes auf der Haut und der dadurch ausgelösten mechanischen Verschiebung bei ausgeführten Körperbewegungen erfolgt eine Reizung der Mechanorezeptoren in der Haut. Dadurch kommt es zur Schmerzdämpfung auf direkter spinaler Ebene.
Der Stand der Technik zu Kinesio weist daraufhin, dass keinerlei Kleber oder ähnliche Substanzen, die in vielen Fällen Unverträglichkeiten oder Allergien auslösen können, im verwendeten Tape enthalten sind.

Ebenso befinden sich keinerlei Wirkstoffe (Arzneien) in den Trägermaterialien und Klebmassen und beeinträchtigen somit weder parallel durchgeführte medikamentöse noch homöopathische Behandlungen.

Beispielsweise ist unter der Marke ChiroTape® ein Kinesiotape bekannt. Hierin führen wellen-förmigen Anhebung der Haut zu einer Druckreduzierung im Gewebe durch Raumvergrößerung zwischen Haut und Muskulatur. Dies hat wiederum eine Regulierung der Lymph- und Blutzirkulation zur Folge.

Zum Unterschied zu Kinesio ist das reine "Tapen" zu verstehen.

WO 2006/ 018 340 A2 beschreibt wirkstoffhaltige tapes zur Behandlung von Gelenkerkrankungen.
"Tapen" ist ein anderer Begriff für "funktionelle Verbandtechnik". Mit diesem Begriff werden Verbände beschrieben, die durch Teilimmobilisierung von Gelenken helfen, gewünschte Bewegungen auszuführen und andere (schmerzhafte) Bewegungen vermeiden. Der Begriff leitet sich vom amerikanischen Begriff "Tape" für Verbandpflaster ab.

Taping hat einerseits zum Ziel, die Kapsel-Band-Strukturen gezielt nachzubilden und dadurch eine selektive Unterstützung und Stabilisierung zu erreichen und andererseits auch eine fördernde Wirkung auf den Heilungsprozess zu erzielen indem Tapeverbände ständig zu Zug- und Druckbelastungen oberflächlicher und tieferliegender Muskelpartien führen. Dadurch werden Anspannung und Durchblutung dieser Muskelpartien erhöht, die Heilung wird beschleunigt, der Muskelabbau vermieden. Die Muskelpumpe kann weiterhin für den Abtransport von Stoffwechselprodukten durch Venen und Lymphgefässe sorgen. In den Gelenken wird das Knorpelgewebe durch die weiterhin mögliche Be- und Entlastung in seiner Funktion erhalten.

Taping stellt heute eine seit vielen Jahren bewährte und durch zahlreiche Untersuchungen belegte Versorgungsmethode zur Prophylaxe und Therapie von Verletzungen, Krankheiten und Veränderungen am Bewegungsapparat dar. Es stützt, schützt und entlastet eine Funktionseinheit, stellt aber keinen Ersatz für eine totale Immobilisation dar, sondern ist vielmehr durch eine gezielte Ruhigstellung nur der verletzten Struktur indiziert bei allen Verletzungen oder Veränderungen, die nicht der völligen Immobilisierung bedürfen.

Der eigentliche Tapeverband wird dabei streifenweise aus vorzugsweise unelastischen selbstklebenden Bändern, so genannten Zügeln, oder in Verbindung mit kurzzugelastischen selbstklebenden Bändern angelegt. Er schützt, stützt und entlastet gefährdete, geschädigte oder gestörte Anteile einer Funktionseinheit. Er erlaubt die selektive Belastung im schmerzfreien Bewegungsraum, verhindert aber extreme oder schmerzhafte Bewegungen.

Das Anlegen derartiger Verbände erfordert fachmännisches Können und Erfahrung und kann deshalb in aller Regel nicht von Laien ohne Taping -Erfahrung ausgeführt werden.

Das Taping ist von der grundlegenden physiologischen Ausrichtung und Wirkungsweise komplett unterschiedlich zu der Kinesio-Therapie. Im Gegensatz dazu soll beim Kinesio keinerlei Immobilisierung ausgeübt werden, die komplette Beweglichkeit des getapten Bereichs bleibt bei der Kinesiotherapie vollständig erhalten.

Beiden Therapieformen ist jedoch eigen auf Wirkstoffe zu verzichten.

Wünschenswert ist es dennoch Kinesio mit den Vorteilen einer Wirkstoffbehandlung zu verbinden.

Weiterhin wünschenswert ist es insbesondere die Wärmetherapie mit Kinesio so zu kombinieren um den Effekt der ständigen Reizwirkung auf der Haut zu erhöhen.

Die Erfindung ist eine selbstklebende Hautauflage umfassend ein nur in einer Richtung dehnbar und rückstellfähiges Trägermaterial. Auf dem Träger ist eine Klebmasse aufgebracht. Die Klebemasse enthält ein oder mehrere Wirkstoffe, wobei die Klebemasse nicht vollflächig, sondern in streifen- oder wellenform aufgetragen ist, die quer zur Dehnbarkeitsrichtung des Trägermaterials verläuft.

Nicht vollflächig bedeutet, dass der prozentuale Anteil an mit Klebmasse beschichteter Trägerfläche maximal 95% beträgt, insbesondere zwischen 20 und 70%, vorzugsweise 40 bis 60%.

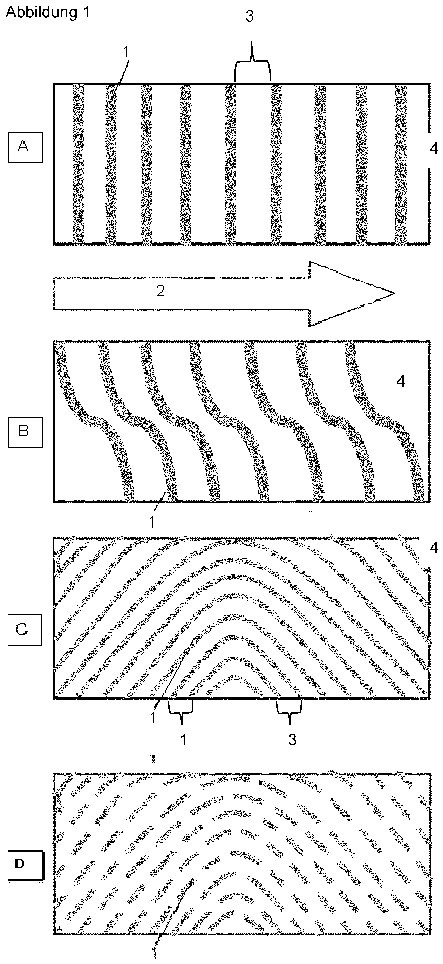

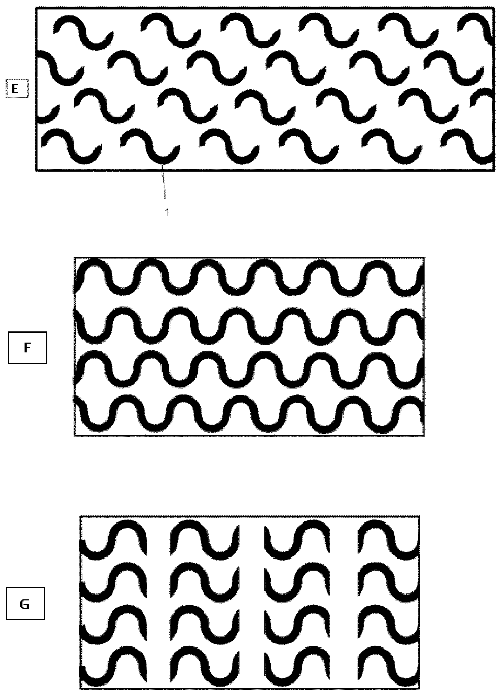

Die nicht beschichteten Bereiche ((3) in Abbildung 1) lassen sich durch den Klebmassenauftrag individuell variieren. Die erfindungsgemäße Kinesio-Wirkstoff-Auflage ermöglicht somit eine Vergrößerung oder Verkleinerung der wellen-förmigen Anhebung der Haut zwischen den nicht beschichteten Bereichen, je nach Anwendungszweck. Die erfindungsgemäße Hautauflage kombiniert die mechanischen Effekten des Kinesio mit den biochemischen Effekten des Wirkstoffes.

Die Klebmasse der erfindungsgemäßen Kinesio-Wirkstoff-Auflage ist auf der Seite des Trägers aufgebracht, so dass beim Applizieren der Auflage die Klebmasse Kontakt mit der Haut aufnehmen kann.

Die Klebmasse ist aufgrund der Kinesioanwendungserfordernis vorteilhaft nicht geschäumt.

In einer bevorzugten Ausführungsform ist die Klebmasse lösemittelhaltig.

Als alternative bevorzugte Ausführungsform ist jedoch auch eine lösemittelfreie Hotmelt (Heißschmelz) -Klebmasse verwendbar. In dieser Ausführungsform kann der Heißschmelzklebstoff, der mit einem Wirkstoff dotiert ist, kontinuierlich mit Hilfe eines Siebdruckverfahrens, oder einer Tiefdruckwalze in die das aufzutragende Muster eingearbeitet ist, aufgebracht werden. Denkbar ist auch die Aufbringung des Heißschmelzklebers in einem diskontinuierlichen Verfahren durch eine, im aufzutragenden Muster angeordneten Anzahl an separaten Düsen. Das Hotmelt Verfahren eignet sich insbesondere bei Strichmustern die von einem einfachen, sinusförmigen Strichbild abweichen.

Bevorzugte Trägermaterialien für erfindungsgemäße selbstklebende Hautauflagen haben eine Dehnbarkeit von 110 bis 200 %, besonders bevorzugt 120 bis 160 % und ganz besonders bevorzugt 130 bis 140 % Dehnbarkeit.

Dehnbarkeit im Sinne der Erfindung bezieht sich auf die Längsrichtung, wobei in Querrichtung das Material weitgehend undehnbar ist. Das Trägermaterial ist in einer Richtung dehnbar, wenn bei einer Kraftaufwendung von 0,05 bis 0,5 N/cm, besonders bevorzugt von 0,1- 0,3 N/cm eine 25%ige Dehnung in Längsrichtung hervorgerufen wird.

Undehnbar bedeutet, dass eine 25%ige Dehnung in Querrichtung erst ab einer Kraft von 80 N/cm, bevorzugt ab 100 N/cm, erzielt wird.

Das Trägermaterial ist in einer Richtung dehnbar und rückstellfähig, wobei sich der erfindungsgemäße streifen -bzw. wellenförmige Klebmassenauftrag quer zu dieser Richtung versteht.

Die Dehnbarkeit ist die relative Längenänderung (Verlängerung bzw. Verkürzung) des Trägers unter Belastung, beispielsweise durch die Bewegungskraft. Wenn die Abmessung des Trägers sich vergrößert, spricht man von einer positiven Dehnung (Streckung), andernfalls von einer negativen Dehnung oder Stauchung.

Die Dehnung (Streckung) der erfindungsgemäßen Träger liegt bei 110 bis 200 %, d.h. sie sind bis auf das Doppelte ihrer Länge verlängerbar ohne zu reißen.

Rückstellfähigkeit bedeutet, dass die gedehnten Materialien in ihre Ausgangslage zurückkehren, wobei je nach Material eine vollständige oder teilweise Rückkehr in den Ausgangszustand erfolgt.

Idealerweise erfolgt nach Dehnung und Wegfall der einwirkenden Kräfte auf das Trägermaterial eine vollständige Rückstellung in den Ausgangszustand.

Das bevorzugte Trägermaterial ist dabei aus einer Naturfaser, wie Baumwolle, Wolle oder einer chemisch modifizierten Naturfaser, wie z.B. Viskose, aufgebaut. Um die Dehnbarkeit oder Rückstellfähigkeit des Trägers zu gewährleisten, und sofern das Gewebe allein die geforderten Dehnungseigenschaften nicht aufweist, enthält das Gewebe des Trägers vorzugsweise zusätzlich einen oder mehrere elastische Fäden, die in die dehnbare Richtung des Materials eingewebt sind. Dieser Faden kann z.B. aus Elastan oder Gummi bestehen. Wobei der elastische Faden wahlweise mit und ohne Ummantelung (durch einen Faden z.B. aus Naturfaser) ausgeführt sein kann. Die Fadenzahlen des Gewebes bewegen sich in Kett- und Schussrichtung im Bereich von 8 - 30 Fäden pro cm. Besonders bevorzugt liegen die Fadenzahlen in Schussrichtung zwischen 15 und 28 und in Kettrichtung zwischen 10 und 20. Ganz besonders bevorzugt liegen die Fadenzahlen in Schussrichtung zwischen 20 und 26 und in Kettrichtung zwischen 12 und 16. Das Flächengewicht der Trägermaterialien liegt dabei üblicherweise im Bereich von 140 - 200 g/m², bevorzugt im Bereich von 150 - 190 g/m², besonders bevorzugt im Bereich von 170 - 185 g/m².

Erfindungsgemäße selbstklebende Hautauflagen können sowohl als Rollenware wie auch als vorgeschnittene Einzelhautauflagen, sogenannte Pre-Cuts, zur Anwendung kommen. Bei beiden Ausführungsformen ist es vorteilhaft wenn die selbstklebenden Hautauflagen mit einer gewissen Vorspannung von 110 bis 160 %, bevorzugt 110 bis 130 % auf das Trennpapier bzw. die Trennfolie, oder bei entsprechender Rollenware auf der eigenen Rückseite kaschiert bzw. geklebt werden.

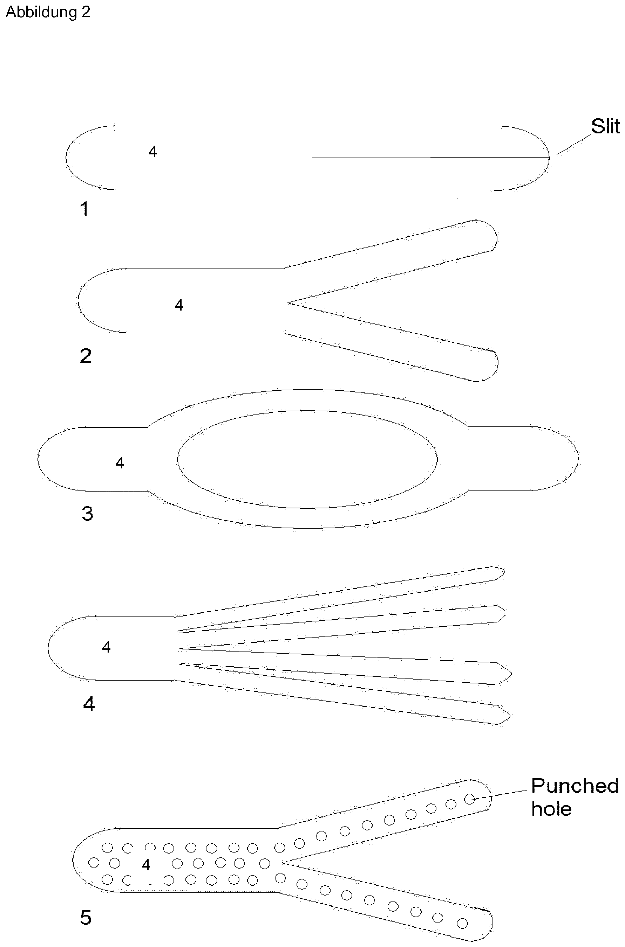

Pre-Cuts können zur Anpassung an jedwede anatomische Gegebenheit vorgeformt sein. Die Produktgeometrien können dabei rund, oval, eckig, regelmäßig und unregelmäßig oder aus Kombinationen davon ausgefertigt sein. In besonderen Ausführungsformen können Pre-Cuts durch entsprechende Veränderungen in der Schichtdicke auch dreidimensional ausgefertigt sein (siehe Abbildung 2).

In der bevorzugten Streifenform haben Pre-Cuts eine maximale Produktbreite von 2 bis 20 cm, bevorzugt 4 bis 6 cm, und eine maximale Produktlänge von 2 bis 100 cm, bevorzugt 5 bis 30 cm, besonders bevorzugt 15 bis 20 cm oder 5-15cm.

Rollenware erfindungsgemäßer selbstklebender Hautauflagen hat ebenfalls eine maximale Produktbreite von 2 bis 20 cm, bevorzugt 4 bis 6 cm, und eine auf Rolle gewickelte Produktlänge von 3 bis 20 m, bevorzugt 5 bis 10 m.

Die Hautauflage wird mit der klebenden Seite auf einem Trennmaterial aufgetragen, das vorteilhaft eine geringere Dehnbarkeit als die Hautauflage aufweist, und als Rolle aufgewickelt wird. Bei der Anwendung kann so eine individuelle Ablängung von der Rolle erfolgen. Zur Anwendung können bedarfsgerechte Längen durch den Anwender selbst abgekürzt werden, wie in den Abbildungen verdeutlicht.

Sowohl die Precuts wie auch die Rollenware sind in einer bevorzugten Ausführungsform nicht auf sich selbst klebend, wie bei klassischen Tapes üblich, sondern mit einem Trennmatrial ausgestattet, das der mit Klebemasse beschichteten Seite zugewandt ist und diese schützt. Das Trennmaterial ist bevorzugt ein Trennpapier, das zu zumindest auf einer Seite mit Silikon oder Flurkohlenwasserstoffen beschichtet ist, oder eine Trennfolie.

Auf die bei Aufrollpflastern und Klebebändern häufig anzutreffende Hydrophobierung des Trägers auf der Oberseite kann in dieser erfindungsgemäß bevorzugten Form der Hautauflage mit Trennmaterial, relase liner, verzichtet werden. Die Hydrophobierung dient dazu ein Selbstverkleben beim Aufrollen zu vermeiden. Erfindungsgemäß wird bevorzugt jedoch ein Trennmaterial (Trennpapier, Trennschicht (release liner)) zwischen Klebschicht und Trägeroberseite eingefügt, so dass es zu keiner Verklebung beim Aufrollen kommen kann.

Die Dehnbarkeit des Trennmaterials ist vorteilhaft geringer als die des Trägers und kann daher bei der Anwendung leicht entfernt werden Der geübte Praktiker kann aufgrund des unterschiedlichen Dehnungsverhaltens des Trägers und des rease liners diesen einreißen und besser entfernen.

In einer weiteren Ausführungsform kann die Auflage zur einfacheren Handhabung mit einem, quer zur Laufrichtung, einreißbaren Trägermaterial versehen sein. Dadurch lässt sich das Tape auch ohne die Zuhilfenahme einer Schere anwenden.

Abbildung 2 zeigt bevorzugte Ausführungsformen, wobei Abbildung 2.1 eine Auflage (4) zur Anwendung an Armen, Beinen, Schulter und Rücken mit einem Einschnitt (slit), Abbildung 2.2 eine Auflage (4) zur Anwendung an Armen, Beinen, Schulter und Rücken, Abbildung 2.3 eine Auflage (4) zur Anwendung an Gelenken z.B. Knien und Abbildung 2.4 eine Auflage (4) zur Anwendung an Armen, Beinen, Schulter und Rücken zeigt.

Die Auflage (4) kann des Weiteren mit einem Lochmuster (punched hole) versehen werden, wie in Abbildung 2.5 dargestellt. Dadurch wird an den Rändern der Löcher ein zusätzlicher Reiz auf die behandelten Hautareale ausgeübt. Die Löcher sind in diesem Fall in mehreren Geraden in Längsrichtung des Tapes angeordnet. Die Lochgröße kann dabei zwischen 3 mm und 8 mm variieren. Der Abstand der Löcher beträgt zwischen 1 cm und 2 Zentimetern.

Gegenstand der vorliegenden Erfindung ist es, die positive Wirkung von Produkten wie Kinesio durch den Einsatz eines hyperämisierenden Wirkstoffes zu steigern.

Die in Tabelle 1 beschriebenen durchblutungsfördernden bevorzugten Substanzen können dazu beitragen Stoffwechselprodukte, die sich z.B. durch Verspannungen in den Muskeln angesammelt haben und den Heilungsprozess behindern, abzutransportieren. Des Weiteren wird durch z.B. Capsaicinoide die Ausschüttung der Substanz P unterdrückt, was zusätzlich Linderung von Schmerzen bringt.

Dabei zeigte sich überraschend, dass die durchblutungsfördernde Wirkung der Capsaicinoide nicht zwangsläufig erforderlich ist, sondern das auch bei Wirkstoffen die lediglich eine Reizwirkung auf die betroffenen Nerven ausüben, schon eine Verstärkung des positiven Kinesio-taping-Effektes erreicht werden kann.

Die Wirkstoffe der erfindungsgemäßen Auflage sind daher nicht auf Capsaicinoide beschränkt. Vorteilhaft ist der Einsatz von Substanzen die die Durchblutung fördern wie z.B. Nicotinsäure Derivate. Die in der nachfolgenden Tabelle aufgeführten Wirkstoffe haben sich dabei als besonders vorteilhaft in der erfindungsgemäßen Kombination aus Wärme- und Kineso-Therapie gezeigt.

**Tabelle 1: Durchblutungsfördernde Wirkstoffe**

| |
|---|
| Capsaicinoide (0,01 - 0,20 %) |
| Nonivamid (0,01 - 0,15 %) |
| Vanilylbutylether (0,5 - 2,5 %) |
| Nicotinsäurebenzylester (0,5 - 4,0 %) |
| Rosmarin Öl (0,5 - 5,0 %) |
| Capsiate (Vanilinsäureester) (0,5 - 2,5 %) |
| Senföle (0,2 - 4,0 %) |

Die o.g. bevorzugten Einsatzkonzentrationen in Gewichtsprozent beziehen sich dabei jeweils auf den Gesamtmassegehalt in der aufgebrachten Klebmasse.

Capsaicin und Nonivamid sind bekannte Wirkstoffe lokal, durchblutungsfördernd wirkender Pflaster. Aufgrund ihrer Anwendung am Bewegungsapparat müssen sie in der Regel stark kleben.

Auch die Klebkraft der erfindungsgemäßen Kinesio-Wirkstoff-Auflagen hat sich als vorteilhaft auf der Haut erwiesen, was beispielsweise zu einer mehrtägige Haltbarkeit der Auflage auf dem Patienten führt.

Beispielsweise zeigen Wirkstoffe wie Vanilylbutylether, deren durchblutungsfördernde Wirkung zwar eher schwach ausgeprägt ist, trotzdem eine Reizwirkung auf die Rezeptoren für Wärme- und Schmerzempfinden. Diese Reizwirkung äußert sich wie bei Capsaicin durch ein wärmendes bis brennendes Gefühl. Dieser Reiz wirkt dem Schmerzreiz durch die z.B. Verspannung der Muskulatur entgegen und führt zu dessen Abschwächung.

Als besonders bevorzugte Wirkstoffe sind hyperämisierende Wirkstoffe zu wählen, insbesondere Wirkstoffe aus der Gruppe der Vanilylakloholderivate, wie z.B. Capsaicinoide oder Ether und Ester des Vanilylalkohols.

Menthol, Campher und deren Derivate, aber auch andere ätherische Öle erniedrigen die Reizschwelle der neuronalen Kälterezeptoren und rufen so ein Kältegefühl hervor. Häufig bewirken sie aber gleichzeitig eine Durchblutungssteigerung, die im Gegenteil ein Wärmegefühl hervorruft. Auch diese Wirkstoffe können in einer bevorzugten Ausführungsform eingesetzt werden.

Weitere zu verwendende Wirkstoffe sind aus der Gruppe der nicht wärmenden, entzündungshemmenden Wirkstoffen aus dem Bereich der Nichtsteroidalen Antientzündlichen Wirkstoffe (engl. NSAIDs) zu wählen. Insbesondere sind dies Aspirin (acetylsalicylic acid), Diflunisal, Salsalate, Ibuprofen, Etofenamat, Dexibuprofen, Naproxen, Fenoprofen, Ketoprofen, Dexketoprofen, Flurbiprofen, Oxaprozin, Loxoprofen, Indomethacin, Tolmetin, Sulindac, Etodolac, Ketorolac, Diclofenac, Nabumetone, Piroxicam, Meloxicam, Tenoxicam, Droxicam, Lornoxicam, Isoxicam, Mefenamic acid, Meclofenamic acid, Flufenamic acid, Tolfenamic acid, Celecoxib , Rofecoxib, Valdecoxib , Parecoxib, Lumiracoxib, Etoricoxib, Firocoxib, Licofelone, Lysine clonixinate, Hyperforin und/oder Figwort. Bevorzugt sind hierbei Ibuprofen, Diclofenac und dessen Salze, Indomethacin, Flurbiprofen und Etofenamat. Dabei kann die Kinesio-Schmerztherapie mit der entzündungs- und damit auch schmerzlindernden medikamentösen Behandlung kombiniert werden, wobei die Einsatzkonzentration in Gewichtsanteilen der Wirkstoffe in der aufgebrachten Klebmasse bevorzugt zwischen 0.01% und 20%, insbesondere zwischen 0.5 und 10% liegt.

Weiterhin möglich ist auch der Einsatz von lokal schmerzunterdrückenden Wirkstoffen (Lokalanesthetika), wie Lidocain, Mepivacain, Prilocain, Articain, Bupivacain, Ropivacain, Etidocain, Dyclonin, Procain, Benzocain, 2-Chlorprocain, Oxybuprocain, Tetracain, Fomocain und ihre jeweiligen Derivate und Salze. Bevorzugt sind insbesondere Lidochain, Prilocain und Benzocain sowie die jeweiligen Derivate/Salze. Dabei kann die Kinesio-Schmerztherapie mit der lokalanästhetischen Behandlung kombiniert werden, wobei die Einsatzkonzentration in Gewichtsanteilen der Wirkstoffe in der aufgebrachten Klebmasse dann zwischen 0.01% und 10%, bevorzugt zwischen 0.3 und 4% liegt.

Ebenfalls einsetzbar sind darüber hinaus geruchsneutrale und /oder kosmetische Wirkstoffe. In diese Gruppe gehören u.a. Cyclohexanecarboxamide (INCI: **Menthane Carboxamide Ethylpyridine**) und 3-((5-Methyl-2-(1-methylethyl)cyclohexyl)oxy)-1,2-propandiol (INCI: Menthoxypropanediol)

Weitere bevorzugte Wirkstoffe sind zu wählen aus der Gruppe Carnitin oder dessen Derivate, Coffein und Capsaicin, insbesondere zwei oder alle drei dieser Wirkstoffe.

Das Verhältnis Carnitin oder dessen Derivate zu Capsaicin und/oder Coffein steht erfindungsgemäß bevorzugt in einem Verhältnis von 1 bis 100 zu 1, vorteilhaft 1 zu 1. D.h. bei einem bevorzugtem Anteil an Carnitin von 0,5 Gew.% hat sich ein Anteil von 0,5 Gew.% Coffein als äußerst wirksam gezeigt. Ebenso vorteilhaft ist dementsprechend ein Verhältnis
von Carnitin, beispielsweise von 0,5 Gew.%, zu Coffein zu 0,3 Gew.% und Capsaicin zu 0,2 Gew.%.

In einer weiteren Ausführungsform kann die Klebmasse der Hautauflage mit hautpflegenden Stoffen, wie z.B. pflanzlichen Ölen, Jojoba, Aloe Vera, oder Liccochalcon A oder Dexpanthenol etc. dotiert sein. Der Vorteil einer solchen Ausführungsform liegt darin, dass Hautreizungen, wie Rötungen etc. durch die zugesetzten pflegenden Substanzen verhindert, zumindest aber abgemildert werden können. Dies ist insbesondere wegen der langen Tragedauer der Kinesio-Auflage sinnvoll.

Bevorzugt umfasst die Klebmasse mehr als einen Wirkstoff, bevorzugt zwei oder drei, insbesondere einen Wirkstoff gewählt aus der Gruppe der hautpflegenden Stoffe und einen bzw. zwei Wirkstoffe gewählt aus der Gruppe der durchblutungsfördernden Stoffe.

Der oder die Wirkstoffe sind bevorzugt zu einem Anteil von 0,01 bis 20 Gew. %, bezogen auf die Gesamtmasse der Klebmasse, enthalten.

Werden Einschränkungen auf bevorzugte Anteilsbereiche einer oder mehrerer Inhaltsstoffe, wie Wirkstoffe, vorgenommen und/oder Einschränkungen auf bestimmte Inhaltsstoffe so beziehen sich die bevorzugten Anteilsbereiche dann auch auf die ausgewählten Inhaltsstoffe.

Die Verteilung der Wirkstoffe in der Klebemasse erfolgt vorzugsweise in einem Thermohomogenisator, wie zum Beispiel Thermomixer, Thermokneter, Walzenwerke oder Schneckensysteme. Die Zugabe des Wirkstoffs kann in die vollständig hergestellte Klebemasse erfolgen. Beispielhaft kann der Wirkstoff auch in eine Zwischenstufe oder in die Ausgangsmischung eingearbeitet werden.

Der Begriff Hautauflage umfasst neben klebenden kosmetisch/medizinischen Pflastern oder Matrices alle kosmetisch/medizinisch anwendbaren Auflagen wie patch, pad, tapes, Tücher, Binden, Dressings, Cataplasmen, Bandagen und/oder Masken.

Selbstklebend bedeutet hierbei, dass die Hautauflage aufgrund der Klebemasse auf der Haut haftet und nicht durch zusätzliche Befestigungsvorrichtungen oder Massen fixiert werden müssen.

Kinesiotapes des Standes der Technik sind normalerweise mit Acrylatklebmassen ausgerüstet. Acrylatklebmassen zeichnen sich durch ein sehr geringes allergenes Potential aus. Darüber hinaus sind sie wasserdampfdurchlässig was dazu führt, dass die Feuchtigkeit, die sich unter dem Verband ansammelt, abdampfen kann. Dieser Umstand begünstigt lange Anwendungsdauern bis hin zu einer Woche, da sich zwischen der Haut und der Klebmasse kein für die Klebkraft schädlicher Feuchtigkeitsfilm mehr ausbilden kann.

Erfindungsgemäße Klebmassen sind vorteilhaft lösemittelbasierte acrylatbasierte Systeme, die nicht einer zusätzlichen Vernetzung durch auf elektromagnetischer Strahlung basierenden Verfahren bedürfen. Eine Ausnahme bilden Systeme die durch Infrarotstrahlung vernetzt werden können. Die Problematik bei der Vernetzung von Acrylatsystemen mit z.B. UV-Strahlung besteht in den sich bildenden Radikalen. Diese hochreaktiven Teilchen können chemische Reaktionen mit den Wirkstoffmolekülen eingehen, wodurch diese entweder völlig zerstört werden oder deren physiologische Wirksamkeit zumindest stark eingeschränkt wird.

Die Acrylatmassen können zusätzliche Stoffe wie zum Beispiel Klebharze, Füllstoffe oder lösliche und unlösliche Hydrokolloide enthalten.

Eine weitere Möglichkeit zur Modifikation der Klebkraft der erfindungsgemäßen Matrices ergibt sich durch die Einstellung der Schichtdicke bzw. der Auftragsmenge der Klebmasse. An sich ist die Klebkraft eine materialspezifische Kenngröße einer Klebmasse und unabhängig von der Schichtdicke. Dies gilt aber nur bis zu einer für die jeweilige Klebmatrix spezifischen Untergrenze der Schichtdicke, unterhalb derer sich die Klebkraft deutlich reduzieren kann.

Die Klebkraft kann, wie im Stand der Technik üblich, auf Polyethylen (PE) gemessen werden. Die so gemessenen Werte sind zwar eine rein technische Beschreibung die nicht 1:1 auf den Menschen zu übertragen ist, jedoch hat sich diese so ermittelte Klebkraft auf PE auch als vorteilhaft für die Haut erwiesen, da die mehrtägige Haltbarkeit des Tapes auf dem Patienten zu ermitteln war.

Beispielhaft sind nachfolgend die Klebkräfte auf Polyethylensubstraten einer lösungsmittelbasierten Acrylatmatrix (Gelva 737 - Vinyl Acetat/Ethyl Acetat) aufgeführt:

**Tabelle 2: Klebkraft auf Polyethylensubstrat**

| Auftragsmenge des Ausstrichs der Klebmatrix | Klebkraft auf Polyethylensubstrat |
|---|---|
| 65 g/m² | 0,722 N/cm |
| 50 g/m² | 0,500 N/cm |
| 42 g/m² | 0,490 N/cm |
| 34 g/m² | 0,351 N/cm |
| 17 g/m² | 0,341 N/cm |

Durch den Zusatz von Lösungsvermittlern wie Ölen und Estern kann die Klebkraft weiterhin modifiziert und somit eine zu hohe Klebkraft reduziert werden. Nachfolgend beispielhaft am Zusatz von 3 % Decyloleat (Cetiol V) in einer lösungsmittelbasierten Acrylatmatrix (Gelva 737) ausgeführt:

**Tabelle 3: Klebkraft auf Polyethylensubstrat mit Lösevermittler**

| Auftragsmenge des Ausstrichs der Klebmatrix plus 3 % Cetiol V | Klebkraft auf Polyethylensubstrat |
|---|---|
| 34 g/m² | 0,095 N/cm |
| 17 g/m² | 0,091 N/cm |

Erfindungsgemäß ist die Klebkraft so eingestellt, dass sie (gemessen auf einem Polyethylensubstrat) bevorzugt zwischen 0,3 bis 0,8 N/cm, besonders bevorzugt zwischen 0,4 und 0,55 N/cm liegt.

Eine weitere bevorzugte Klebmassentype der vorliegenden Erfindung beruht auf lösemittelbasierten Kautschukklebmassen. Besonders bevorzugt sind hier Systeme die auf synthetischen Kautschuken, wie Styrol-Isopren-Styrol Blockcopolymeren oder Olefinmonomeren (z.B. Isopren oder Isobuten) beruhen. Diese Systeme haben den Vorteil, dass sie nicht zusätzlich vernetzt werden müssen, wodurch die eingesetzten Wirkstoffe deutlich schonender verarbeitet werden können.

Wie bei den Acrylatsystemen können auch die Kautschuk basierten Klebmassen mit Füllstoffen, Harzen und Hydrokolloiden versetzt sein.

Bevorzugte Klebmassen umfassen Polyisobutylene, Styrol Butadien Rubber, Styrol Isopren Styrol Blockcopolymere sowie ggf. Zusatzstoffe wie Kohlenwasserstoffharze, Colophoniumglycerolester und/oder pulvrige Cellulose.

Der Einsatz von Hydrokolloiden in löslicher oder unlöslicher Form begünstigt eine lange Tragdauer des Verbandes, da die von der Haut abgegebene Feuchtigkeit absorbiert und zur Außenseite des Verbandes abgeführt werden kann.

Gegenüber den klassischen Acrylatklebemassen kann die Auflage, wie beschrieben, auch mit Kautschukklebmassen beschichtet werden. Diese haben gegenüber vielen Acrylatklebmassen den Vorteil eines aggressiveren Klebverhaltens, was die Anwendungsdauer des Produktes deutlich verlängern kann.

Bevorzugt handelt es sich bei der Klebmasse auch um eine lösemittelfreie hot-melt (Heißschmelz) Klebmasse.

Desweiteren ist die Verwendung Polyurethan- und Silikon-Klebemassen erfindungsgemäß bevorzugt.

Erfindungsgemäß sind die Klebemassen in einer speziellen Ausrichtung aufgebracht, die die mechanische Wirkung der Auflage (4) zusätzlich verstärkt. Es kann beispielsweise ein Streifenstrich (A) oder Wellenstrich (B, C) der Klebmasse (1) zum Einsatz kommen, die in Querrichtung zur Dehnungsfähigkeit (2) des Trägermaterials auf diesem aufgebracht sind, wie es in Abbildung 1 dargestellt ist.

Querrichtung zur Dehnungsfähigkeit des Trägers ist dabei erfindungsgemäß so zu verstehen, dass mehr als 90% der Klebmassenstreifen nicht parallel zur Dehnungsrichtung des Trägers verlaufen.

90% bezieht sich dabei auf die Gesamtmasse an aufgetragener Klebmasse.

In diesem Sinne sind in Abbildung 1C lediglich die Wellenberge des Klebmassenauftrags als parallel zur Dehnungsrichtung des Trägers orientiert anzusehen. Der überwiegende Anteil (>90%) der Klebmasse ist quer, also nicht parallel (Winkel 5 ≠ 0°), zur Dehnungsrichtung des Trägers aufgetragen. Als quer zur Dehnungsrichtung des Trägermaterials werden alle diejenigen Klebmassenstreifen oder-wellen verstanden, deren Winkel (5) größer als 5° zur Dehnungsrichtung des Trägers betragen. Der Anteil an Klebmassenstreifen oderwellenbereichen, die einen Winkel von 5° oder kleiner zur Dehnungsrichtung des Trägers aufweisen, ist erfindungsgemäß kleiner 10% zur insgesamt aufgetragenen Klebmasse.

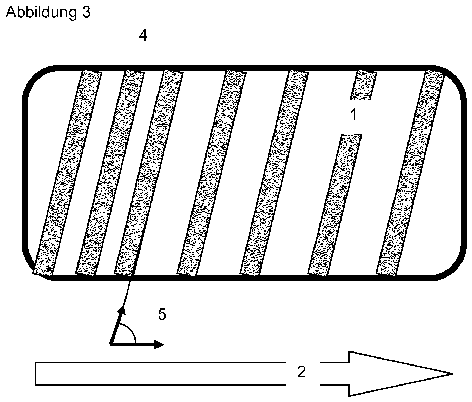

Erfindungsgemäß bevorzugt ist der Klebmassenauftrag in Streifenform aufgetragen und die Streifen (1) weisen einen Winkel (5) zur Dehnungsrichtung des Trägers (2) im Bereich von 45° bis 90° auf, insbesondere 90°(s. Abbildung 3).

Eine partielle Beschichtung von Trägermaterialien mit druckempfindlichen Heißschmelzklebern (PSA-Hotmelts - PSA = Pressure Sensitive Adhesives) ist eine bekannte Technik, sei es, dass die Hotmeltmasse beispielsweise durch Thermosiebdruck, wie beispielsweise in DE 42 37 252 A1 beschrieben oder durch Raster- oder Tiefdruck von in Längs- oder Querrichtung zu zusammenhängenden Stegen aufgebracht wird (DE 43 08 649 A1). Hierbei wird das Trägermaterial nur teilweise vom Klebefilm bedeckt. Offene Systeme lassen an den offenen Stellen des Klebefilms bzw. des Trägermaterials, also den Bereichen des Trägermaterials die nicht mit Klebmasse bedeckt sind, einen guten Feuchtigkeitsaustausch zu.

Wird ein wellenförmiger Klebmassenauftrag vorgesehen, so ist es von Vorteil wenn die Wellenbeschichtung mit einer Amplitude größer 20 mm Höhe erfolgt. Diese gegenüber dem Stand der Technik (z.B. DE 10012582 A1) höheren Amplitudenwerte sind für den erfindungsgemäßen Anwendungszweck vorteilhaft, da sie zu größeren Bewegungen der Haut führen.

Der Vorteil der streifen- bzw. wellenförmigen Auftragung des Trägermaterials liegt in der unterbrochenen Verklebung mit der Haut. An den nicht mit Klebmasse beschichteten Stellen bilden sich temporäre Hautfalten die zu einer Art "Mikromassage" des betroffenen Areals führen. Die Hautfalten resultieren aus der Vordehnung des Trägers und der Verklebung. Durch die Bewegung des Körpers und der Muskeln werden die klebenden Teile des Pflasters gegen die nicht klebenden, mit Hautfalten versehenen Stellen verschoben. Diese Verschiebung führt zu einem andauernden Reiz der sich in die unter der Haut liegenden Muskelschichten fortsetzt. Dieser andauernde Reiz trägt erheblich zur Heilung bei. Der Heilungseffekt wird durch die eingesetzten Wirkstoffe erfindungsgemäß jedoch wesentlich verstärkt.

Die Anordnung der Streifen oder Wellen quer zur Dehnungsrichtung ermöglicht auch erst einen Feuchtigkeitsabtransport, da die nicht mit Klebmasse beschichteten Areale sich bis zum Rand des Pflasters fortpflanzen. Das ermöglicht, neben der Verdampfung durch die unbeschichteten Teile des Trägermaterials, ein Abführen der Feuchtigkeit in Bereiche außerhalb der mit der Auflage beklebten Fläche, weil die Ränder der Auflage nicht mehr vollständig mit Klebmasse "versiegelt" sind.

Die Abführung von Flüssigkeiten von der Hautoberfläche ist durch die nicht beschichteten Bereiche des Pflasters sichergestellt, da hier eine nahezu ungehinderte Wasserdampfdurchlässigkeit gewährleistet ist.

Ein Gittermuster als Anordnung erhöht zwar die Klebkraft führt aber dazu, dass die Ausbildung der zuvor beschriebenen Hautfalten eingeschränkt wird. Durch diesen Effekt wird ebenfalls die Ausbildung des beschriebenen Reizes abgeschwächt, da sich allenfalls noch punktförmige Falten ausbilden können. Diese Gebilde können aber auf Grund ihrer geometrischen Form einer mechanischen Beanspruchung leichter ausweichen. Zudem verhindert ein Gitterauftrag der Klebmasse den erfindungsgemäßen Vorteil des Flüssigkeitstransportes zum Rand hin.

Der erfindungsgemäße streifen- oder wellenförmige Klebmassenauftrag quer zur Dehnungsrichtung des Trägers ist erfindungsgemäß bevorzugt so gestaltet, dass der Bereich zwischen zwei benachbarten Klebmassenstreifen zu einem Rand der Auflage offen ist (3) (s. Abbildung 1 C), bevorzugt zu zwei Rändern der Auflage (siehe Abbildung 1A und B).

Die Freisetzung der Wirkstoffe ist im Produkt vorteilhaft so eingestellt, dass auch nach einem Zeitraum von 2 Tagen noch eine Wirkung wahrgenommen werden kann. Die retardierte Wirkung der eingesetzten Wirkstoffe wird dabei aus Kombination von Wirkstoffgehalt und Hilfsstoffen erreicht. Über den Einsatz von z.B. Weichmachern und Füllstoffen lässt sich die Freisetzung der Wirkstoffe über einen weiten zeitlichen Bereich steuern. Beispiele für solche Systeme sind auf dem Markt befindliche Schmerzpflaster mit Opiaten wie Fentanyl, die über mehrere Tage bis hin zu einer Woche angewendet werden und eine absolut gleichmäßige Freisetzung des Wirkstoffs über den gesamten Anwendungszeitraum gewährleisten. Für die Bestimmung der Freisetzungsrate von Vanillylbutylether (VBE) aus z.B. Acrylatmassen wurden dieses mit 0,75% in die Klebmassen eingearbeitet und die Freisetzung über die Zeit in Schweinehaut mittels Franz'scher Zellen ermittelt. Die Bestimmung der freigesetzen Mengen in die Vollhaut erfolgte danach per HPLC.

**Tabelle 4:Wirkstofffreisetzung VBE**

| Acrylatmatrix | Prozentuale Freisetzung VBE nach 24 h |
|---|---|
| Gelva 737 | 2,0 |
| DuroTak 129 | 2,7 |

Eine mögliche Maßnahme zur Erhöhung der Wirkstofffreisetzung ist der Einsatz von Lösungsvermittlern. Erfindungsgemäß bevorzugt wird Decyloleat (Cetiol V), Octyldodecanol und eine Ölmischung aus Olivenöl, Arganöl und Sheabutter verwendet. Durch die Zugabe von Cetiol V konnte eine signifikante Zunahme der freigesetzten Menge VBE in Schweinehaut erreicht werden, teilweise bis zur Verdoppelung der freigesetzten Menge. Für die beiden anderen Additive wurden geringere Werte ermittelt, so dass insbesondere Decyloleat (Cetiol V) bevorzugter Lösungsvermittler in der Klebmasse ist.

**Tabelle 5: Wirkstofffreisetzung VBE mit Lösevermittler nach 24h**

| Acrylatmatrix | Prozentuale Freisetzung VBE nach 24 h |
|---|---|
| Gelva 737 + 3% Cetiol V | 4,0 |
| DuroTak 129 + 3% Cetiol V | 3,5 |

Bedingt durch die lange Tragdauer des Kinesio-Auflage von mehreren Tagen ist auch die Kenntnis der Freisetzungskinetik über einen längeren Zeitraum entscheidend. Auf Schweinehaut ist dies durch die natürlichen Zersetzungsprozesse der Haut nicht möglich und wurde daher auf einer Silikonfolie durchgeführt. Dabei wurde jeden Tag eine Probe der Rezeptorphase aus der Franz-Zelle entnommen und diese in der HPLC vermessen.

**Tabelle 6: Wirkstofffreisetzung VBE mit Lösevermittler nach 7d**

| Acrylatmatrix | Prozentuale Freisetzung VBE nach 7 d |
|---|---|
| Gelva 737 + 3% Cetiol V | 43,0 |
| DuroTak 129 + 3% Cetiol V | 41,0 |

Die relativ hohen Werte ergeben sich daher, dass der Wirkstoff leicht durch die dünne Silikonfolie in die Rezeptorphase diffundieren kann. Für beide Acrylatmassen zeigt sich der gleiche Verlauf in der Freisetzung über die Applikationsdauer. In den ersten 24 Stunden erfolgt die höchste Freisetzung an VBE, welche über die Zeit auf ein Plateau zuläuft. Entscheidend ist, dass über die gesamte Zeitspanne geringe Mengen freigesetzt werden.

Zur Bestimmung der Freisetzungsrate von Capsaicin aus einer Acrylatmasse (Gelva 737) wurde ebenfalls Cetiol V als Lösungsvermittler genutzt. Hierzu wurde 0,355% Capsaicin in die Masse eingearbeitet.

**Tabelle 7: Wirkstofffreisetzung Capsaicin nach 24h**

| Matrix | Prozentuale Freisetzung Capsaicin nach 24 h |
|---|---|
| Gelva 737 | 0,4 |

Mit 0,4% nach 24 Stunden penetriert nur relativ wenig Capsaicin aus der Gelva 737 in die Haut. Wie schon beim VBE wurde auch bei Capsaicin die Freisetzung über 7 Tage untersucht. Hierbei wurde, zusätzlich zur Gelva 737 Duro-Tak 129 in die Betrachtungen mit einbezogen. In der folgenden Tabelle sind die Summen an freigesetztem Capsaicin angegeben.

**Tabelle 8: Wirkstofffreisetzung Capsaicin nach 7d**

| Matrix | Prozentuale Freisetzung Capsaicin nach 7 d |
|---|---|
| Gelva 737 | 37,0 |
| DuroTak 129 | 31,0 |

Die Acrylatmassen untereinander zeigen einen sehr ähnlichen Verlauf, so dass sich beide Massen hinsichtlich der Capsaicinfreisetzung als geeignet erweisen. Wie schon bei VBE wird auch hier über die gesamte Zeitspanne Wirkstoff in geringen Mengen freigesetzt.

Die erfindungsgemäßen Hautauflagen lassen sich zur kombinierten Wirkstoff - und Kinesiotherapie in der Gelenk-, Muskel- und/oder Hautbehandlung sowohl therapeutisch als auch nicht-therapeutisch verwenden.

Gelenk-, Muskel- und/oder Hautbehandlung ist dabei insbesondere die physiotherapeutische Behandlung des menschliches Skeletts, Muskulatur und Hautareale.

Als therapeutisches Verfahren sind die Hautauflagen zur Herstellung eines Behandlungsmittels für die Behandlung des menschliches Skeletts, Muskulatur und Hautareale zu verwenden.

Nachfolgende Beispiele veranschaulichen die erfindungsgemäßen selbstklebenden Hautauflagen.

### Beispiel 1:

Man legt 53,04 g Oppanol B80, 106,08 g Oppanol B10 SFN, 53,04 g SBR 1011AC, 79,56 g Vector 4113 und 2,64 g Lowinox 22 M 46 in 500 g Benzin (70 - 90°C) einem Dispenser vor. Die Mischung wird 3 Stunden bei Raumtemperatur mit 2500 U/min dispergiert und anschließend über Nacht ruhen gelassen. Am nächsten Tag gibt man weitere 100 g Benzin (70 - 90°C) hinzu, fügt 98,10 g Regalite 7100 und 100,74 g Staybelite Ester 10 zu. Die Mischung wird dann 2,5 Stunden bei Raumtemperatur mit einer Umdrehungszahl von 2500 U/min dispergiert. Anschließend gibt man 21,24 g Vitacel L600/30 zu und dispergiert weitere 1,5 Stunden mit 2500 U/min bei Raumtemperatur. Abschließend werden 79,56 g Sheabutter und 6,00 g Vanilylbutylether zugegeben. Die Klebmasse wird bei 2500 U/min für weitere 30 min dispergiert.

Die so erhaltene Klebmasse wird dann mit einer handelsüblichen Streichvorrichtung auf ein Trägermaterial in Streifenform ausgestrichen und getrocknet.

### Beispiel 2:

In einem Z-Blatt Kneter werden 20,00 g Oppanol B 80 vorgelegt und bei einer Vorlauftemperatur von 150 °C krümelig geknetet. Anschließend gibt man 53,40 g Oppanol B10 SFN und 46,00 g Vector 4113 zu. Die Mischung wird dann 10 min homogenisiert. Nach der Homogenisierungsphase gibt man 28,00 g Regalite 7100 und 12,60 g Staybelite Ester 10 zu. Es folgt eine Homogenisierungsphase von 150 min bei einer Vorlauftemperatur von 100°C. Zu der so erhaltenen Mischung werden 8,00 g Vitacel L600/30 zugegeben und über einen Zeitraum von 120 min homogenisiert. Abschließend gibt man 12,50 g Olivenöl, 6,00 g Arganöl, 12,40 g Sheabutter und 0,10g Capsaicin zu. Die Mischung wird weitere 60 min bei einer Vorlauftemperatur von 90°C homogenisiert.

Die fertige Klebmasse wird dann mittels einer Hydraulikpresse bei 90°C und einem Druck von 200 bar ausgepresst. Anschließend wir das Trägermaterial zukaschiert.

Zur technischen Routineproduktion kann die Rezeptur auch mittels Extruder hergestellt werden bzw. per Transportschnecke und Schlitzdüse auf ein Substrat aufgebracht und anschließend mit dem Trägermaterial kaschiert werden. Je nach maschinellen Gegebenheiten kann die Matrix aber auch zunächst auf das Trägermaterial verstrichen, geschnitten und aufgerollt bzw. mit Trennpapier kaschiert, geschnitten und aufgerollt werden.

Der erfindungsgemäß vorteilhafte Streifen- oder Wellenstrich kann dabei durch maschinell einstellbare Lateralbewegungen des Substrats quer zur Laufrichtung einfach erzeugt werden.

### Beispiel 3:

In einem Dispenser werden 500g Acrylatklebmasse (Gelva 737-01 in Ethylacetat) mit einem Feststoffgehalt von 34 % vorgelegt. Zu dieser Klebmasse gibt man 0,17g Capsaicinoide und rührt die Mischung für einen Zeitraum von 30 min. Die Klebmasse wird anschließend auf silikonisiertem Papier ausgestrichen und nach der Trocknung mit einem geeigneten Trägermaterial eingedeckt.

Verwendete Rohstoffe und Handelsnamen:

| | |
|---|---|
| Oppanol B 10 SFN | Polyisobutylen |
| Oppanol B 80 | Polyisobutylen |
| SBR 1011 AC | Styrol Butadien Rubber |
| Vector 4113 | Styrol Isopren Styrol Blockcopolymer |
| Lowinox 22 M 46 | Alterungsschutz (Phenol, 2,2'-methylenebis[6-(1,1-dimethylethyl)-4-methyl-2,2'-Methylenebis(6-*t*-butyl-4-methylphenol)) |
| Regalite 7100 | Kohlenwasserstoffharz (Partially Hydrogenated Hydrocarbon Resins) |
| Staybelite Ester 10 | Colophoniumglycerolester |
| Vitacel L600/30 | pulvrige Cellulose |

## Patentansprüche

1. Selbstklebende Hautauflage umfassend ein nur in einer Richtung dehnbar und rückstellfähiges Trägermaterial und eine auf dem Träger nicht vollflächig aufgebrachte Klebemasse enthaltend ein oder mehrere Wirkstoffe aus der Gruppe der durchblutungsfördernden Wirkstoffe, wobei die Klebemasse in Streifen- oder Wellenform aufgetragen ist, die quer zur Dehnbarkeitsrichtung des Trägermaterials verläuft, was so zu verstehen ist, dass mehr als 90% der Klebmassenstreifen, bezogen auf die Gesamtmasse an aufgetragener Klebmasse, nicht parallel zur Dehnungsrichtung des Trägermaterials verlaufen.

2. Hautauflage nach Anspruch 1 **dadurch gekennzeichnet, dass** die Klebmasse lösemittelhaltig ist.

3. Hautauflage nach Anspruch 1 **dadurch gekennzeichnet, dass** die Klebmasse eine lösemittelfreie hot-melt Klebmasse ist.

4. Hautauflage nach einem der vorstehenden Ansprüche **dadurch gekennzeichnet, dass** Vanilylbutylether als Wirkstoff enthalten ist.

5. Hautauflage nach einem der vorstehenden Ansprüche **dadurch gekennzeichnet, dass** als Wirkstoff Carnitin oder dessen Derivate, Coffein und/oder Capsaicin, insbesondere zwei oder alle drei Wirkstoffe, enthalten sind.

6. Hautauflage nach Anspruch 5 **dadurch gekennzeichnet, dass** das Gewichtsverhältnis Carnitin oder dessen Derivate zu Capsaicin und/oder Coffein im Bereich von 1 bis 100 zu 1, vorteilhaft 1 zu 1, zu wählen ist.

7. Hautauflage nach einem der vorstehenden Ansprüche **dadurch gekennzeichnet, dass** der oder die Wirkstoffe zu einem Anteil von 0,01 bis 20 Gew. %, bezogen auf die Gesamtmasse der Klebmasse, enthalten sind.

8. Hautauflage nach einem der vorstehenden Ansprüche **dadurch gekennzeichnet, dass** ein oder mehrere Lösungsvermittlern in der Klebmasse enthalten sind, insbesondere Decyloleat.

9. Hautauflage nach einem der vorstehenden Ansprüche **dadurch gekennzeichnet, dass** das Trägermaterial eine Dehnbarkeit von 110 bis 200% aufweist.

10. Hautauflage nach Anspruch 9 **dadurch gekennzeichnet, dass** das Trägermaterial ein Gewebe umfasst in den ein oder mehrere elastische Fäden in die dehnbare Richtung des Materials eingewebt ist.

11. Hautauflage nach einem der vorstehenden Ansprüche **dadurch gekennzeichnet, dass** der Klebmassenauftrag in Streifenform aufgetragen ist und die Streifen (1) einen Winkel (5) zur Dehnungsrichtung des Trägers (2) im Bereich von 45° bis 90° aufweisen.

12. Hautauflage nach einem der vorstehenden Ansprüche mit einer Klebkraft gemessen auf einem Polyethylensubstrat zwischen 0,3 bis 0,8 N/cm, besonders bevorzugt zwischen 0,4 und 0,55 N/cm, liegt.

13. Hautauflage nach einem der vorstehenden Ansprüche **dadurch gekennzeichnet, dass** die Klebmasse nicht geschäumt ist.

14. Hautauflage nach einem der vorstehenden Ansprüche **dadurch gekennzeichnet, dass** die Klebmasse in Wellenform aufgetragen ist mit einer Amplitude des Wellenauftrags größer 20 mm.

15. Hautauflage nach einem der vorstehenden Ansprüche **dadurch gekennzeichnet, dass** das Flächengewicht der Trägermaterialien im Bereich von 140 - 200 g/m², bevorzugt im Bereich von 150 - 190 g/m², besonders bevorzugt im Bereich von 170 - 185 g/m², liegt.

16. Verwendung der Hautauflagen nach einem der vorstehenden Ansprüche 1 bis 15 zur Herstellung eines Behandlungsmittels für die Behandlung des menschliches Skeletts, Muskulatur und/oder Hautareale.

17. Verwendung der Hautauflage nach einem der vorstehenden Ansprüche 1 bis 15 zur individuellen Ablängbarkeit **dadurch gekennzeichnet, dass** die Hautauflage mit der klebenden Seite auf einem Trennmaterial, das eine geringe Dehnbarkeit als die Hautauflage aufweist, aufgetragen und insgesamt als Rolle gewickelt ist.

## Claims

1. Self-adhesive dressing comprising a support material that is stretchable and reboundable only in one direction, and an adhesive mass not applied to the support over the whole area and comprising one or more active substances from the group of the circulation-promoting active substances, where the adhesive mass is applied in stripe form or wave form which runs crossways to the stretchable direction of the support material, which is to be understood as meaning that more than 90% of the adhesive mass stripes, based on the total mass of applied adhesive mass, do not run parallel to the direction of stretching of the support material.

2. Dressing according to Claim 1, **characterized in that** the adhesive mass is solvent-containing.

3. Dressing according to Claim 1, **characterized in that** the adhesive mass is a solvent-free hot-melt adhesive mass.

4. Dressing according to any one of the preceding claims, **characterized in that** vanilyl butyl ether is present as active substance.

5. Dressing according to any one of the preceding claims, **characterized in that** the active substance present is carnitine or derivatives thereof, caffeine and/or capsaicin, in particular two or all three active substances.

6. Dressing according to Claim 5, **characterized in that** the weight ratio of carnitine or derivatives thereof to capsaicin and/or caffeine is to be selected in the range from 1 to 100:1, advantageously 1:1.

7. Dressing according to any one of the preceding claims, **characterized in that** the active substance or active substances are present in an amount of from 0.01 to 20% by weight, based on the total mass of the adhesive mass.

8. Dressing according to any one of the preceding claims, **characterized in that** one or more solubility promoters are present in the adhesive mass, in particular decyl oleate.

9. Dressing according to any one of the preceding claims, **characterized in that** the support material has a stretchability of from 110 to 200%.

10. Dressing according to Claim 9, **characterized in that** the support material comprises a fabric into which one or more elastic threads are woven in the stretchable direction of the material.

11. Dressing according to any one of the preceding claims, **characterized in that** the adhesive mass application is applied in stripe form and the stripes (1) have an angle (5) relative to the stretching direction of the support (2) in the range from 45° to 90°.

12. Dressing according to any one of the preceding claims with an adhesive force, measured on a polyethylene substrate, between 0.3 and 0.8 N/cm, particularly preferably between 0.4 and 0.55 N/cm.

13. Dressing according to any one of the preceding claims, **characterized in that** the adhesive mass is not foamed.

14. Dressing according to any one of the preceding claims, **characterized in that** the adhesive mass is applied in wave form with an amplitude of the wave application greater than 20 mm.

15. Dressing according to any one of the preceding claims, **characterized in that** the areal weight of the support materials is in the range from 140-200 g/m², preferably in the range from 150-190 g/m², particularly preferably in the range from 170-185 g/m².

16. Use of the dressings according to any one of the preceding Claims 1 to 15 for producing a treatment agent for treating the human skeleton, musculature and/or skin areas.

17. Use of the dressings according to any one of the preceding Claims 1 to 15 for the individual ability to be cut into lengths, **characterized in that** the dressing is applied with the sticky side to a release material, which has a lower stretchability than the dressing, and is wound overall as a roll.

## Revendications

1. Pansement autoadhésif comprenant un matériau support extensible et élastique uniquement dans une direction et une masse adhésive non appliquée sur l'ensemble de la surface du support, contenant un ou plusieurs agents actifs du groupe des agents actifs favorisant la circulation sanguine, la masse adhésive étant appliquée sous la forme de bandes ou d'ondulations qui sont perpendiculaires à la direction d'extensibilité du matériau support, ce qui signifie que plus de 90 % des bandes de masse adhésive, par rapport à la masse totale de masse adhésive appliquée, ne sont pas parallèles à la direction d'extension du matériau support.

2. Pansement selon la revendication 1, **caractérisé en ce que** la masse adhésive contient un solvant.

3. Pansement selon la revendication 1, **caractérisé en ce que** la masse adhésive est une masse adhésive thermofusible sans solvant.

4. Pansement selon l'une quelconque des revendications précédentes, **caractérisé en ce que** de l'éther de vanilylbutyle est contenu en tant qu'agent actif.

5. Pansement selon l'une quelconque des revendications précédentes, **caractérisé en ce que** de la carnitine ou ses dérivés, de la caféine et/ou de la capsaïcine, notamment deux ou les trois agents actifs, sont contenus en tant qu'agent actif.

6. Pansement selon la revendication 5, **caractérisé en ce que** le rapport en poids entre la carnitine ou ses dérivés et la capsaïcine et/ou la caféine est choisi dans la plage allant de 1 à 100 sur 1, avantageusement de 1 sur 1.

7. Pansement selon l'une quelconque des revendications précédentes, **caractérisé en ce que** le ou les agents actifs sont contenus en une proportion de 0,01 à 20 % en poids, par rapport à la masse totale de la masse adhésive.

8. Pansement selon l'une quelconque des revendications précédentes, **caractérisé en ce qu'**un ou plusieurs solubilisants sont contenus dans la masse adhésive, notamment de l'oléate de décyle.

9. Pansement selon l'une quelconque des revendications précédentes, **caractérisé en ce que** le matériau support présente une extensibilité de 110 à 200 %.

10. Pansement selon la revendication 9, **caractérisé en ce que** le matériau support comprend un tissu dans lequel un ou plusieurs fils élastique sont incorporés dans la direction extensible du matériau.

11. Pansement selon l'une quelconque des revendications précédentes, **caractérisé en ce que** la masse adhésive est appliquée sous la forme de bandes et les bandes (1) présentent un angle (5) par rapport à la direction d'extension du support (2) dans la plage allant de 45° à 90°.

12. Pansement selon l'une quelconque des revendications précédentes ayant une force d'adhérence mesurée sur un substrat en polyéthylène comprise entre 0,3 et 0,8 N/cm, de manière particulièrement préférée entre 0,4 et 0,55 N/cm.

13. Pansement selon l'une quelconque des revendications précédentes, **caractérisé en ce que** la masse adhésive n'est pas moussée.

14. Pansement selon l'une quelconque des revendications précédentes, **caractérisé en ce que** la masse adhésive est appliquée sous la forme d'ondulations avec une amplitude des ondulations appliquées supérieure à 20 mm.

15. Pansement selon l'une quelconque des revendications précédentes, **caractérisé en ce que** le poids superficiel des matériaux supports se situe dans la plage allant de 140 à 200 g/m², de préférence dans la plage allant de 150 à 190 g/m², de manière particulièrement préférée dans la plage allant de 170 à 185 g/m².

16. Utilisation des pansements selon l'une quelconque des revendications 1 à 15 précédentes pour la fabrication d'un agent de traitement pour le traitement du squelette, de la musculature et/ou de zones de la peau humaine.

17. Utilisation des pansements selon l'une quelconque des revendications 1 à 15 précédentes pour la découpe individuelle, **caractérisée en ce que** le pansement est appliqué avec le côté adhésif sur un matériau anti-adhérent, qui présente une plus faible extensibilité que le pansement, et ils sont enroulés ensemble sous la forme d'un rouleau.
